# EUROPEAN PATENT APPLICATION

(11) **EP 2 818 470 A1**
(43) Date of publication of application: **31.12.2014**
(21) Application number: 13174050.8
(22) Date of filing: 27.06.2013
(51) Int. Cl.: C07D 413/12, A61K 31/513, A61P 31/18

(54) **Cocrystals of raltegravir potassium**

(71) Applicant: BASF SE, 67056 Ludwigshafen (DE)
(72) Inventor: Chiodo, Tiziana, Dr., 68159 Mannheim (DE); Hafner, Andreas, 4460 Gelterkinden (CH); Hintermann, Tobias, Dr., 4106 Therwil (CH); Vossen, Marcus, Dr., 67117 Limburgerhof (DE); Salvador, Beate, 67158 Ellerstadt (DE); Weishaar, Walter, 67269 Grünstadt (DE); Viertelhaus, Martin, Dr., 68259 Mannheim (DE)

(57) **Abstract**

Novel solid forms of Raltegravir potassium comprising the active ingredient N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide as potassium salt co-crystal with cyclamic acid, saccharin, stearic acid or succinic acid. The multi-component crystalline forms show improved properties such as dissolution kinetic and hydration stability.

## Description

The present invention relates to multicomponent systems comprising raltegravir potassium and selected co-crystal formers, to pharmaceutical preparations comprising said systems, and specifically to homogenous crystalline phases (cocrystals) comprising raltegravir potassium and selected co-crystal formers. The invention also relates to processes for preparing said multicomponent systems and crystalline phases. The invention also relates to compositions comprising said multicomponent systems or crystalline phases and pharmaceutically acceptable carrier, and to methods of using said multicomponent systems or crystalline phases to treat HIV infections.

Raltegravir is the synonym of the name: N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide (due to its enolic structure also known as 4-fluorobenzyl-aminocarbonyl-5-hydroxy-6-oxo-1-methyl-2-(1-methyl-1-([(5-methyl-1,3,4-oxadiazol-2-yl)carbonyl]amino)ethyl)-1,6-dihydropyrimidine), specifically shown in formula (1)

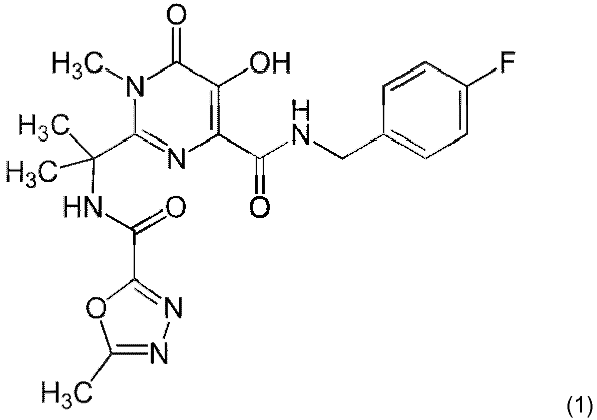

It is known to act on the integrase, an HIV enzyme that integrates the viral genetic ma-terial into human chromosomes, a critical step in the pathogenesis of HIV. Raltegravir is useful in the treatment of HIV infections; its marketed product Isentress® contains raltegravir as potassium salt:

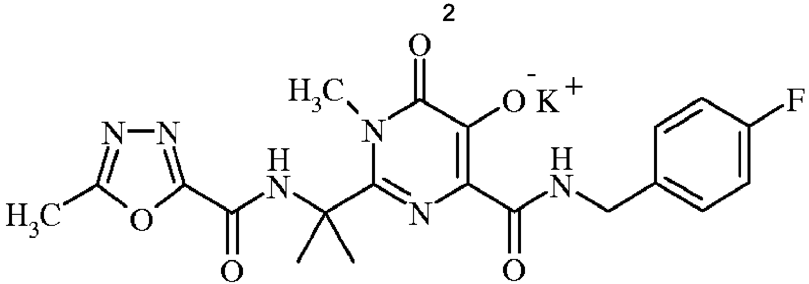

In WO 06/ 060712 are disclosed two crystalline forms of raltegravir potassium.

In WO 10/ 140156 are disclosed an anhydrous and a hydrate crystalline forms of raltegravir potassium, hereafter referred to as form 1 and form H1.

In WO 11/0241924 are disclosed an amorphous and two crystalline forms (referred to as form A and form B) of raltegravir free hydroxy potassium. Three crystalline form of raltegravir potassium are disclosed and hereafter referred to as form A, form B and form C.

WO 11/ 067236 discloses further crystalline forms of ralregravir free hydroxy, hereafter referred to as form 1 and form 2.

WO 11/ 123754 discloses crystalline forms of raltegravir salts with potassium (16 pcrystalline forms), sodium (3 crystalline forms), lithium, calcium, tert-buthylamine, diethylamine, diisopropylamine.

In EP 2522665 are disclosed further crystalline forms of raltegravir potassium, hereafter referred to as form A to form H .

WO 12/137142 discloses novel raltegravir salts with meglumine, erbumine, ammonium, tris, L-arginine.

Existing solid forms of raltegravir and its salts leave room for improvement of physical as well as biological characteristics. There exists a need for other solid forms, especially crystalline forms, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt for sufficient diversity on crystalline materials to optimize manufacture, formulation and biological efficiency and to control its complex polymorphism.

### Summary of the Invention:

The invention provides the description of novel crystalline forms of raltegravir potassium salt comprising raltegravir potassium and selected cocrystal formers, and processes for manufacture thereof.

One of the said crystalline forms shows desired different physical and/or biological characteristics which may assist in the manufacture or formulation of the active compound, to the purity levels and uniformity required for regulatory approval. The said crystalline form may possess improved pharmacological characteristics, for example, improved bioavailability, thus offering enhanced possibilities to modulate and design improved drug products.

### Detailed Description of the Invention:

The solid form of the invention is a composite comprising two components, which are raltegravir potassium and a pharmaceutical co-crystal former (in the following also denoted as coformer). In this invention, the pharmaceutical co-crystal formers contained with raltegravir potassium within one single anhydrous phase are: cyclamic acid, saccharin, stearic acid or succinic acid:

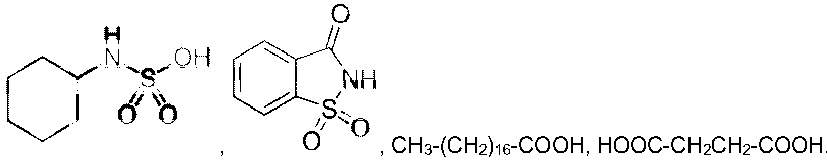

Raltegravir potassium and cyclamic acid or saccharin or stearic acid or succinic acid are present in the same solid phase, preferably in the same crystalline phase, i.e. forming a co-crystal. Preferred cocrystals are those of Raltegravir potassium and either cyclamic acid or stearic acid.

The molar ratio of raltegravir potassium and and and cyclamic acid or saccharin or stearic acid or succinic acid is generally in the range from 2:1 to 1:2, in particular from 1.5:1 to 1:1.5 and especially about 1:1 (i.e. from 1.1:1 to 1:1.1).

The invention thus includes
a) a multicomponent molecular crystal containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and cyclamic acid; and more specifically a1) an anydrous crystalline form as defined under a) consisting essentially of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and cyclamic acid, having stoichiometric ratio from 2:1 to 1:2.

Preferred solid form defined under (a1) may be further characterized by its melting point of above 203°C (m.p. typically within the range 203 - 213°C, especially 206 - 210 °C; each as determined by DSC).

The present solid form provides better dissolution characteristic and hygroscopic behavior, when compared with the raltegravir potassium forms previously known.

The melting point of the present cocrystal form contrasts to the melting point of the anhydrous Raltegravir potassium (crystalline form A of WO06060712) used as the present starting material, which is 279 °C.

Raltegravir potassium and cyclamic acid are present in the same solid phase, preferably in the same crystalline phase, i.e. forming a co-crystal. The preferred novel crystalline form generally exhibits a characteristic X-ray powder diffraction pattern with characteristic peaks expressed in d-values (Å): 16.77 (vs), 13.89 (m), 6.95 (m), 4.21 (m).

More specifically, the present invention comprises a crystalline form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and cyclamic acid, having stoichiometric ratio from 2:1 to 1:2, which exhibits a characteristic X-ray powder diffraction pattern with characteristic peaks expressed in d-values (Å) as shown in the below table:

| d value | 2θ value | Intensity |
|---|---|---|
| [Angstroem] | [°] | |
| 16.77 | 5.27 | vs |
| 13.89 | 6.36 | m |
| 10.68 | 8.28 | w |
| 8.07 | 10.96 | w |
| 7.48 | 11.84 | w |
| 6.95 | 12.72 | m |
| 6.43 | 13.77 | w |
| 5.69 | 15.57 | w |
| 5.34 | 16.59 | w |
| 4.21 | 21.12 | m |

Here and in the following the abbreviations in brackets mean: (vs) = very strong intensity; (s) = strong intensity; (m) = medium intensity; (w) = weak intensity.
b) a multicomponent molecular crystal containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and saccharin; and more specifically
b1) an anydrous crystalline form as defined under b) consisting essentially of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and saccharin, having stoichiometric ratio is from 2:1 to 1:2.

Preferred solid form defined under b may be further characterized by its melting point of above 242°C (m.p. e.g. from the range 242 - 252°C, especially from the range 245-249 °C . The present solid form provides better dissolution characteristic and hygroscopic behavior compared to raltegravir potassium forms previously known.

Raltegravir potassium and saccharin are present in the same solid phase, preferably in the same crystalline phase, i.e. forming a co-crystal. The preferred novel crystalline form generally exhibits a characteristic X-ray powder diffraction pattern with characteristic peaks expressed in d-values (Å): 14.52 (vs), 13.93 (s), 10.70 (m), 6.96 (s).

More specifically, the present invention comprises a crystalline form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and saccharin, having stoichiometric ratio from 2:1 to 1:2, which exhibits a characteristic X-ray powder diffraction pattern with characteristic peaks expressed in d-values (Å) as shown in the below table:

| d value | 2θ value | Intensity |
|---|---|---|
| [Angstroem] | [°] | |
| 14.52 | 6.09 | Vs |
| 13.93 | 6.34 | S |
| 10.70 | 8.26 | M |
| 8.10 | 10.92 | W |
| 7.52 | 11.77 | W |
| 6.96 | 12.72 | S |
| 6.42 | 13.79 | W |
| 4.47 | 19.88 | W |
| 4.40 | 20.19 | W |
| 4.10 | 21.65 | W |

Here and in the following the abbreviations in brackets mean: (vs) = very strong intensity; (s) = strong intensity; (m) = medium intensity; (w) = weak intensity.
c) a multicomponent molecular crystal containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and stearic acid; and more specifically
c1) an anydrous crystalline form as defined under c) consisting essentially of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and stearic acid, having stoichiometric ratio is from 2:1 to 1:2.

Preferred solid form defined under i may be further characterized by its melting point of above 102°C (m.p. e.g. from the range 102 - 112°C, especially from the range 105-109 °C). The present solid form provides better dissolution characteristic and hygroscopic behavior, when compared with the raltegravir potassium forms previously known.

Raltegravir potassium and stearic acid are present in the same solid phase, preferably in the same crystalline phase, i.e. forming a co-crystal. The preferred novel crystalline form generally exhibits a characteristic X-ray powder diffraction pattern with characteristic peaks expressed in d-values (Å): 24.63 (s), 16.59 (vs), 15.39 (m), 9.99 (m).

More specifically, the present invention comprises a crystalline form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and stearic acid, having stoichiometric ratio from 2:1 to 1:2, which exhibits a characteristic X-ray powder diffraction pattern with characteristic peaks expressed in d-values (Å) as shown in the below table:

| d value | 2θ value | Intensity |
|---|---|---|
| [Angstroem] | [°] | |
| 24.63 | 3.59 | s |
| 16.59 | 5.33 | vs |
| 15.39 | 5.74 | m |
| 12.48 | 7.08 | w |
| 9.99 | 8.85 | m |
| 8.37 | 10.57 | w |
| 7.16 | 12.37 | w |
| 5.59 | 15.84 | w |
| 5.20 | 17.05 | w |
| 4.54 | 19.56 | w |

Here and in the following the abbreviations in brackets mean: (vs) = very strong intensity; (s) = strong intensity; (m) = medium intensity; (w) = weak intensity.
d) a multicomponent molecular crystal containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and succinic acid; and more specifically
d1) an anydrous crystalline form as defined under a) consisting essentially of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and succinic acid, having stoichiometric ratio is from 2:1 to 1:2.

Preferred solid form defined under d may be further characterized by its melting point of above 244°C (m.p. e.g. from the range 244 - 254°C, especially from the range 247-251 °C . The present solid form provides better dissolution characteristic and hygroscopic behavior, when compared with the raltegravir potassium forms previously known.

Raltegravir potassium and succinic acid are present in the same solid phase, preferably in the same crystalline phase, i.e. forming a co-crystal. The preferred novel crystalline form generally exhibits a characteristic X-ray powder diffraction pattern with characteristic peaks expressed in d-values (Å): 13.83 (vs), 10.64 (s), 9.16 (s), 6.94 (vs), 5.34 (m), 4.39 (m).

More specifically, the present invention comprises a crystalline form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and succinic acid, having stoichiometric ratio 1:1, which exhibits a characteristic X-ray powder diffraction pattern with characteristic peaks expressed in d-values (Å) as shown in the below table:

| d value | 2θ value | Intensity |
|---|---|---|
| [Angstroem] | [°] | |
| 13.83 | 6.39 | vs |
| 10.64 | 8.31 | s |
| 9.16 | 9.66 | s |
| 7.93 | 11.16 | w |
| 6.94 | 12.76 | vs |
| 6.40 | 13.83 | w |
| 5.69 | 15.59 | w |
| 5.34 | 16.61 | m |
| 4.46 | 19.91 | w |
| 4.39 | 20.22 | m |

Here and in the following the abbreviations in brackets mean: (vs) = very strong intensity; (s) = strong intensity; (m) = medium intensity; (w) = weak intensity.

Another object of the invention is a process for the preparation of a crystalline form as defined above which comprises the steps of
a) combining raltegravir potassium and the co-crystal former in a suitable solvent,
b) agitating the mixture obtained in step (a), and
c) separating the solid material and drying.

The solvent used in step (a) is suitably selected according to the solubility of raltegravir potassium and of the co-crystal former. Solvent according to step (a) preferably is a solvent or solvent system wherein each of the components raltegravir potassium and the co-crystal former have a similar solubility. Thus, step (a) typically leads, at least in part, to a dissolution of each of the components (herein recalled as suspension in case that no complete dissolution is effected). Step (a) may result in a suspension of the 2 components, or in a suspension of one component in a solution of the other component, or in a solution of both components; preferred is a solution of both components, and especially a suspension of both components.

The concentration of raltegravir potassium salt in step (a) may typically range from 0.1 to about 300 mg/ml of solvent (including water), preferably from 20 to 200 mg/ml.

The process is preferably carried out in the temperature range 15-50°C, for example at ambient temperature. In a preferred process, step (c) is carried out at a temperature from the range 30-60°C or the mixture is heated to a temperature from said range, e.g. about 50°C, especially in case that solid raltegravir potassium is provided in step (a), with forming a solution. The solution thus tempered is then preferably cooled before step (c), i.e. before separation.

Ambient temperature means in the context of the invention a temperature range from room temperature to about 30°C, comprising e.g. 20 to 30 °C and preferably about 23 to 26 °C.

The cocrystal defined under a, b, c and d is isolated e.g. by decantation of the liquid, centrifugation and/or filtering off the crystals, which are subsequently dried, e.g. in vacuum, inert gas flow or both, typically at ambient temperature or elevated temperatures up to 80°C.

Cocrystals of the invention enable to improve the dissolution characteristics of raltegravir potassium, i.e. providing a better dissolution kinetic profile with respect to the previously known raltegravir potassium.

Cocrystals defined under a, b, c and d are thermodynamically stable and can be dried at elevated temperatures, e.g. below 80°C, and are obtained as a fine powder with typical particle size distributions with the median size between 1 and 50 µm, preferably between 1 to 10 µm. This particle size range ensures a fast dissolution profile, while retaining the favourable handling properties in the formulation process.

Cocrystal defined under a, b, c and d are less prone to water uptake under humidity, and is easy to formulate. Cocrystal defined under a, b, c and d generally contains minor amounts of water, mainly within its crystal structure, the amounts usually ranging from 1.5 to 5% of water, relative to the total weight of the solid phase.

Cocrystals of the present invention may be used in pharmaceutical compositions in the same way as other forms of raltegravir potassium previously known.

The present invention further pertains to a pharmaceutical composition comprising the crystalline material or multicomponent molecular crystal described above, comprising N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and cyclamic acid, saccharin, stearic acid or succinic acid as a coformer, and a pharmaceutically acceptable carrier or diluent. Preferred is a pharmaceutical composition comprising N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and the coformer within the same crystalline phase,
characterized in that
the coformer is cyclamic acid, and the composition exhibits at least one X-ray powder diffraction peak, expressed in d-values (Å), selected from the group consisting of 16.77, 13.89, 6.95, 4.21;
or the coformer is saccharin, and the composition exhibits at least one X-ray powder diffraction peak, expressed in d-values (Å), selected from the group consisting of 14.52, 13.93, 10.70, 6.96;
or the coformer is stearic acid, and the composition exhibits at least one X-ray powder diffraction peak, expressed in d-values (Å), selected from the group consisting of 24.63, 16.59, 15.39, 9.99;
or the coformer is succinic acid, and the composition exhibits at least one X-ray powder diffraction peak, expressed in d-values (Å), selected from the group consisting of 13.83, 10.64, 9.16, 6.94, 5.34, 4.39.

The present invention is also directed to a pharmaceutical composition comprising a solid form containing cyclamic acid, or especially crystal form defined under a, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and hydrates thereof, and a pharmaceutically acceptable carrier or diluent.

The present invention is also directed to a pharmaceutical composition comprising a solid form containing saccharin, or especially crystal form defined under b , of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and hydrates thereof, and a pharmaceutically acceptable carrier or diluent.

The present invention is also directed to a pharmaceutical composition comprising a solid form containing stearic acid, or especially crystal form defined under c , of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and hydrates thereof, and a pharmaceutically acceptable carrier or diluent.

The present invention is also directed to a pharmaceutical composition comprising a solid form containing succinic acid, or especially crystal form defined under d , of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and hydrates thereof, and a pharmaceutically acceptable carrier or diluent.

The amount of solid (especially crystalline) forms of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, or hydrates thereof, substantially depends on type of formulation and desired dosages during administration time periods. The amount in an oral formulation may be, for example, from 0.1 to 1000 mg, preferably from 1 to 500 mg, and more preferably from 5 to 400 mg.

Oral formulations may be solid formulations such as capsules, tablets, pills and troches, or liquid formulations such as aqueous suspensions, elixirs and syrups. Solid and liquid formulations encompass also incorporation of the present solid form, especially co-crystal forms a, b, c and/or d, into liquid or solid food.

The solid forms according to the invention may be directly used as powders (micronized particles), granules, suspensions or solutions, or they may be combined together with other pharmaceutically acceptable ingredients in admixing the components and optionally finely divide them, and then filling capsules, composed for example from hard or soft gelatine, compressing tablets, pills or troches, or suspend or dissolve them in carriers for suspensions, elixirs and syrups. Coatings may be applied after compression to form pills.

Pharmaceutically acceptable ingredients are well known for the various types of formulation and may be for example binders such as natural or synthetic polymers, excipients, lubricants, surfactants, sweetening and flavouring agents, coating materials, preservatives, dyes, thickeners, adjuvants, antimicrobial agents and carriers for the various formulation types.

Examples for binders are gum tragacanth, acacia, starch, gelatine, and biological degradable polymers such as homo- or co-polyesters of dicarboxylic acids, alkylene glycols, polyalkylene glycols and/or aliphatic hydroxyl carboxylic acids; homo- or copolyamides of dicarboxylic acids, alkylene diamines, and/or aliphatic amino carboxylic acids; corresponding polyester-polyamide-co-polymers, polyanhydrides, polyortho-esters, polyphosphazene and polycarbonates. The biological degradable polymers may be linear, branched or crosslinked. Specific examples are poly-glycolic acid, poly-lactic acid, and poly-d,l-lactide/glycolide. Other examples for polymers are water-soluble polymers such as polyoxaalkylenes (polyoxaethylene, polyoxapropylene and mixed polymers thereof, poly-acrylamides and hydroxylalkylated polyacrylamides, poly-maleic acid and esters or -amides thereof, poly-acrylic acid and esters or -amides thereof, poly-vinylalcohol und esters or -ethers thereof, poly-vinylimidazole, poly-vinylpyrrolidon, und natural polymers like chitosan, carragenan or hyaluronic aid.

### Examples for excipients are phosphates such as dicalcium phosphate.

Examples for lubricants are natural or synthetic oils, fats, waxes, or fatty acid salts like magnesium stearate.

Surfactants may be anionic, anionic, amphoteric or neutral. Examples for surfactants are lecithin, phospholipids, octyl sulfate, decyl sulfate, dodecyl sulfate, tetradecyl sulfate, hexadecyl sulfate and octadecyl sulfate, Na oleate or Na caprate, 1-acylaminoethane-2-sulfonic acids, such as 1-octanoylaminoethane-2-sulfonic acid, 1-decanoylaminoethane-2-sulfonic acid, 1-dodecanoylaminoethane-2-sulfonic acid, 1-tetradecanoylaminoethane-2-sulfonic acid, 1-hexadecanoylaminoethane-2-sulfonic acid, and 1-octadecanoylaminoethane-2-sulfonic acid, and taurocholic acid and tauro-deoxycholic acid, bile acids and their salts, such as cholic acid, deoxycholic acid and sodium glycocholates, sodium caprate or sodium laurate, sodium oleate, sodium lauryl sulphate, sodium cetyl sulphate, sulfated castor oil and sodium dioctylsulfosuccinate, cocamidopropylbetaine and laurylbetaine, fatty alcohols, cholesterols, glycerol mono-or -distearate, glycerol mono- or -dioleate and glycerol mono- or -dipalmitate, and poly-oxyethylene stearate.

Examples for sweetening agents are sucrose, fructose, lactose or aspartam.

Examples for flavouring agents are peppermint, oil of wintergreen or fruit flavours like cherry or orange flavour.

Examples for coating materials gelatine, wax, shellac, sugar or biological degradable polymers.

Examples for preservatives are methyl or propylparabens, sorbic acid, chlorobutanol, phenol and thimerosal.

Examples for adjuvants are fragrances.

Examples for thickeners are synthetic polymers, fatty acids and fatty acid salts and esters and fatty alcohols.

Examples for liquid carriers are water, alcohols such as ethanol, glycerol, propylene glycol, liquid polyethylene glycols, triacetin and oils. Examples for solid carriers are talc, clay, microcrystalline cellulose, silica, alumina and the like.

The formulation according to the invention may also contain isotonic agents, such as sugars, buffers or sodium chloride.

The solid forms according to the invention may also be formulated as effervescent tablet or powder, which disintegrate in an aqueous environment to provide a drinking solution.

A syrup or elixir may contain the polymorph of the invention, sucrose or fructose as sweetening agent a preservative like methylparaben, a dye and a flavouring agent.

The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage form and prepared by any of the methods well-known in the art of pharmacy.

Dosage forms include solid dosage forms, like tablets, powders, capsules, suppositories, sachets, troches and losenges as well as liquid suspensions and elixirs. While the description is not intended to be limiting, the invention is also not intended to pertain to true solutions of raltegravir, or its potassium salt, whereupon the properties that distinguish the solid forms of the invention are lost. However, the use of the novel forms to prepare such solutions is considered to be within the contemplation of the invention.

Capsule dosages, of course, will contain the solid composition within a capsule which may be made of gelatin or other conventional encapsulating material. Tablets and powders may be coated. Tablets and powders may be coated with an enteric coating. The enteric coated powder forms may have coatings comprising phthalic acid cellulose acetate, hydroxypropylmethyl-cellulose phthalate, polyvinyl alcohol phthalate, carbox-ymethylethylcellulose, a copolymer of styrene and maleic acid, a copolymer of meth-acrylic acid and methyl methacrylate, and like materials, and if desired, they may be employed with suitable plasticizers and/or extending agents. A coated tablet may have a coating on the surface of the tablet or may be a tablet comprising a powder or granules with an enteric-coating.

Slow release formulations may also be prepared from the crystal form according to the invention in order to achieve a controlled release of the active agent in contact with the body fluids in the gastro intestinal tract, and to provide a substantial constant and effective level of the active agent in the blood plasma. The crystal forms may be embedded for this purpose in a polymer matrix of a biological degradable polymer, a water-soluble polymer or a mixture of both, and optionally suitable surfactants. Embedding can mean in this context the incorporation of micro-particles in a matrix of polymers. Controlled release formulations are also obtained through encapsulation of dispersed micro-particles or emulsified micro-droplets via known dispersion or emulsion coating technologies.

In a preferred embodiment, the cocrystal defined under a, b, c, or d of the invention is administered orally in a pharmaceutical composition comprising the cocrystal and hydroxypropylmethylcellulose (e.g., HPMC 2910), wherein the composition is compressed into a tablet. In another preferred embodiment, the cocrystal defined under a, b, c, or d of the invention is administered orally in a pharmaceutical composition comprising the cocrystal, a poloxamer (e.g., poloxamer 407), hydroxypropylmethylcellulose (e.g., HPMC 2208), and lactose (e.g., lactose monohydrate), wherein the composition is compressed into a tablet.

The crystal forms of the invention are also useful for administering a combination of therapeutic effective agents to an animal. Such a combination therapy can be carried out in using at least one further therapeutic agent which can be additionally dispersed or dissolved in a formulation.

The crystal forms of this invention and its formulations respectively can be also administered in combination with other therapeutic agents that are effective to treat a given condition to provide a combination therapy.

The present invention includes administration of an effective amount of either of cocrystals defined above under a, b, c and d (either alone or as the active component of a pharmaceutical composition) for inhibiting HIV integrase, for the treatment or prophylaxis of HIV infection, or for the treatment, prophylaxis, or delay in the onset of AIDS to a subject in need of such inhibition, treatment, prophylaxis, or delay. The present invention also includes the use of a cocrystals defined above under a, b, c and d in combination with an anti-HIV agent.The term "administration" and variants thereof (e.g., "administering" a compound) in reference to a cocrystal defined under a, b, c, or d of the invention mean providing the salt to the individual in need of inhibition, treatment or prophylaxis. When a cocrystal defined under a, b, c, or d of the invention is provided in combination with one or more other active agents (e.g., antiviral agents useful for treating or prophylaxis of HIV infec-tion or AIDS), "administration" and its variants are each understood to include provision of the compound or prodrug and other agents at the same time (separately or together)
or at different times.

The term "anti-HIV agent" means an agent which is effective in one or more of the following uses: inhibiting integrase or another enzyme required for HIV replication or infection, prophylaxis of HIV infection, treating HIV infection, delaying the onset of AIDS, prophylaxis of AIDS, or treating AIDS. Suitable anti-HIV agents include HIV/AIDS antiviral agents, anti-infective agents, and immunomodulators.

The crystalline forms of the invention may be used as single component or as mixtures with other solid forms, which may be crystalline or amorphous.

As to the novel crystalline forms of raltegravir potassium it is preferred that these contain 25-100% by weight, especially 50-100% by weight, of at least one of the novel forms, based on the total amount of raltegravir potassium. Preferably, such an amount of the novel polymorphic forms of raltegravir potassium is 75-100% by weight, especially 90-100% by weight. Highly preferred are forms consisting essentially of one or more of the present crystalline forms; the term "consisting essentially of" here and elsewhere in this text means an amount of 95-100% by weight.

Another object of the invention is a method of delivering a solid form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and/or hydrates thereof to a host, which method comprises administering to a host an effective amount of said solid form, especially crystal form a, b, c and/or d, according to the invention.

A further object of the invention is the use of a crystal form a and/or solid form containing cyclamic acid, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, or hydrates thereof, for the manufacture of a medicament useful for inhibiting HIV integrase, for the treatment or prophylaxis of HIV infection, or for the treatment, prophylaxis, or delay in the onset of AIDS to a subject in need of such inhibition, treatment, prophylaxis, or delay. Especially for use in a mammal, such as a human; and the solid forms according to the invention for use in medical therapy. The present invention also includes the use of a cocrystals defined above under a in combination with an anti-HIV agent. A further object of the invention is the use of a crystal form b and/or solid form contain-ing saccharine, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, and hydrates thereof, for the manufacture of a medicament useful for inhibiting HIV integrase, for the treatment or prophylaxis of HIV infection, or for the treatment, prophylaxis, or delay in the onset of AIDS to a subject in need of such inhibi-tion, treatment, prophylaxis, or delay. Especially for use in a mammal, such as a hu-man; and the solid forms according to the invention for use in medical therapy. The present invention also includes the use of a cocrystals defined above under b in com-bination with an anti-HIV agent.

A further object of the invention is the use of a crystal form c and/or solid form containing stearic acid, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, and hydrates thereof, for the manufacture of a medicament useful for inhibiting HIV integrase, for the treatment or prophylaxis of HIV infection, or for the treatment, prophylaxis, or delay in the onset of AIDS to a subject in need of such inhibition, treatment, prophylaxis, or delay. Especially for use in a mammal, such as a human; and the solid forms according to the invention for use in medical therapy. The present invention also includes the use of a cocrystals defined above under c in combination with an anti-HIV agent.

A further object of the invention is the use of a crystal form d and/or solid form containing succinic acid, of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, and hydrates thereof, for the manufacture of a medicament useful for inhibiting HIV integrase, for the treatment or prophylaxis of HIV infection, or for the treatment, prophylaxis, or delay in the onset of AIDS to a subject in need of such inhibition, treatment, prophylaxis, or delay. Especially for use in a mammal, such as a human; and the solid forms according to the invention for use in medical therapy. The present invention also includes the use of a cocrystals defined above under d in combination with an anti-HIV agent

The following examples illustrate the invention.

Wherever noted, room temperature depicts a temperature from the range 18-23°C; percentages are given by weight, if not indicated otherwise.

Abbreviations used in in the examples or in the above specification:

| | |
|---|---|
| Å | the length 10⁻¹⁰ m (Angstroem) |
| HPLC | high pressure liquid chromatography |
| NMR | nuclear magnetic resonance |
| FTIR | Fourier-transformation infrared spectrometry |
| DSC | Differential scanning calorimetry |
| r.h. | relative humidity (air, if not indicated otherwise) |
| TG | thermogravimetry |
| v/v | volume by volume |

### Instrumental

Powder X-ray diffraction (PXRD) is carried out with a Panalytical X'Pert Pro X-ray diffractometer using Cu_{K-alpha} radiation in reflection (Bragg-Brentano) geometry. 2θ values are accurate within an error of ±0.1-0.2°. The samples are prepared without any special treatment other than the application of slight pressure to get a flat surface. The tube voltage is 40 kV, current is 40 mA. The XRPD diffractograms are collected at room temperature in the geometry in the range from 2θ =3°-35°C with increments of 0.0167°C.

Thermogravimetry: The thermogravimetric measurements are carried out with a Mettler Toledo TGA/SDTA851e module. The thermal behaviour is analysed in the range 30-250°C by using a heating rate of 5°C/min and a stream of nitrogen flowing at 150 ml/ during the experiment.

DSC is performed on a Mettler Toledo DSC 822e module. The sample is placed in crimped but vented aluminium pans (sample size was 10 mg). The thermal behaviour is analysed in the range 30 - 250°C by using a heating rate of 5°C/min and a stream of nitrogen flowing at 150 ml/ during the experiment.

1 H-NMR: The 1 H-NMR spectra are recorded on a Bruker DPX 300 spectrometer. Solvent: dmso-d6.

### Experimental

Solvents: For all experiments, Fluka or Merck grade solvents are used.

### Educts:

N-Cyclohexylsulfamic acid (Aldrich - 29550-500G, purum, min 98,0%);
Saccharin (Fluka - 12475, purum, > 99,0%);
Stearic acid (Sigma - 17.536-6, Reagent grade, min. 95%);
Succinic acid (Merck - 8.22260.0250, min. 99%);
Raltegravir potassium: Anhydrous crystalline form as of WO06060712.

### Crystallization experiments:

The crystallization experiments are performed on a Crystalline device. The glass vials containing the crystallization solutions are stirred using magnetic stirrers and are placed into a temperature controlled chamber. The applied cooling rate is 1 K/min. The solutions are cooled until -20 °C .

### Example 1: Preparation of the co-crystal comprising raltegravir potassium salt and cyclamic acid.

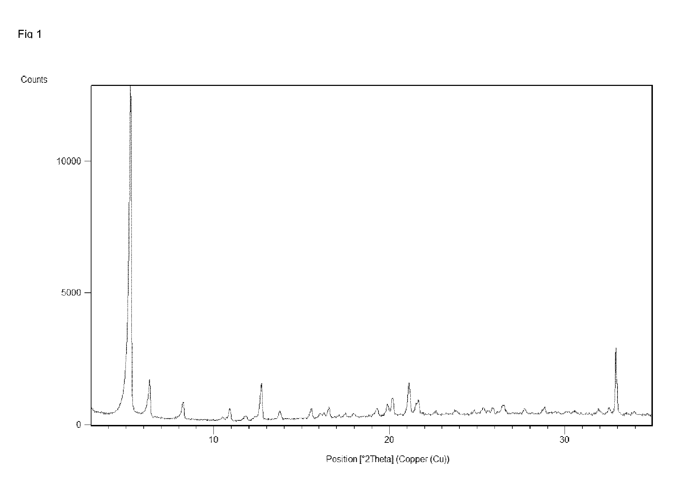

73 mg of raltegravir potassium salt and 27 mg of cyclamic acid are suspended in 3 ml of isopropanol. The suspension is stirred at 25 °C for 24 hours. The obtained solid is filtered and air dried overnight. XRPD of the dried sample shows the pattern shown in Figure 1, corresponding to a co-crystal comprising raltegravir potassium and cyclamic acid. DSC confirms the formation of a pure phase (m.p. 208°C) and TG does not show any mass loss, indicating the formation of an anhydrous phase. 1 H-NMR (measured in dmso-d6) shows the spectrum of a mixture of raltegravir and cyclamic acid acid. Elemental analysis confirm the expected amount of potassium.

### Example 2: Preparation of the co-crystal comprising raltegravir potassium salt and saccharin.

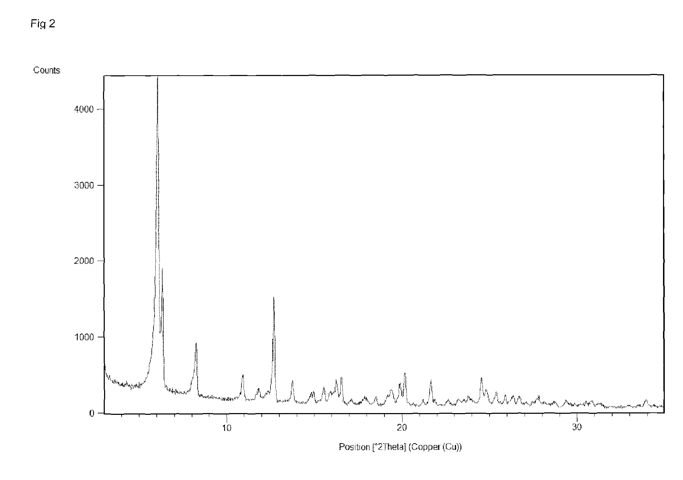

73 mg of raltegravir potassium salt and 27 mg of saccharin are suspended in 3 ml of ethyl acetate. The suspension is stirred at 25 °C for 24 hours. The obtained solid is filtered and air dried overnight. XRPD of the dried sample shows the pattern shown in Figure 2, corresponding to a co-crystal comprising raltegravir potassium and saccharin. DSC confirms the formation of a pure phase (m.p. 247°C) and TG does not show any mass loss, indicating the formation of an anhydrous phase. 1 H-NMR (measured in dmso-d6) shows the spectrum of a mixture of raltegravir and saccharin. Elemental analysis confirm the expected amount of potassium.

### Example 3: Preparation of the co-crystal comprising raltegravir potassium salt and stearic acid.

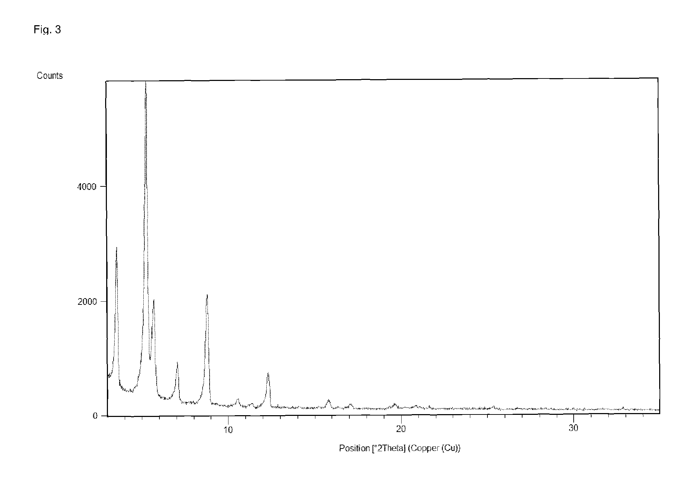

63 mg of raltegravir potassium salt and 37 mg of stearic acid are suspended in 3 ml of acetone. The suspension is stirred at 25 °C for 24 hours. The obtained solid is filtered and air dried overnight. XRPD of the dried sample shows the pattern shown in Figure 3, corresponding to a co-crystal comprising raltegravir potassium and stearic acid. DSC confirms the formation of a pure phase (m.p.107°C) and TG does not show any mass loss, indicating the formation of an anhydrous phase. 1 H-NMR (measured in dmso-d6) shows the spectrum of a mixture of raltegravir and stearic acid acid. Elemental analysis confirm the expected amount of potassium.

### Example 4: Preparation of the co-crystal comprising raltegravir potassium salt and succinic acid.

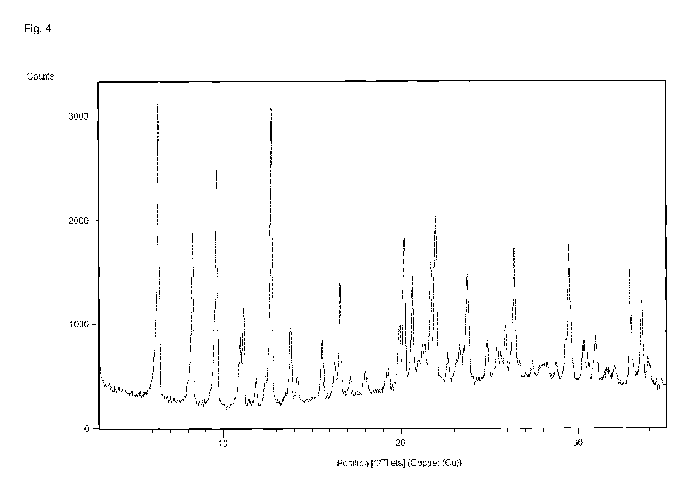

80 mg of raltegravir potassium salt and 20 mg of succinic acid are suspended in 3 ml of ethyl acetate. The suspension is stirred at 25 °C for 24 hours. The obtained solid is filtered and air dried overnight. XRPD of the dried sample shows the pattern shown in Figure 4, corresponding to a co-crystal comprising raltegravir potassium and succinic acid. DSC confirms the formation of a pure phase (m.p. 249°C) and TG does not show any mass loss, indicating the formation of an anhydrous phase. 1 H-NMR (measured in dmso-d6) shows the spectrum of a mixture of raltegravir and succinic acid acid. Elemental analysis confirm the expected amount of potassium.

Brief description of Figures:
**Figure 1****:** Powder X-Ray Diffraction pattern of raltegravir potassium and cyclamic acid co-crystal
**Figure 2****:** Powder X-Ray Diffraction pattern of raltegravir potassium and saccharin co-crystal
**Figure 3****:** Powder X-Ray Diffraction pattern of raltegravir potassium and stearic acid co-crystal
**Figure 4****:** Powder X-Ray Diffraction pattern of raltegravir potassium and succinic acid co-crystal

## Claims

1. A crystalline material comprising N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and a coformer selected from the group consisting of cyclamic acid, stearic acid, saccharin, and succinic acid, especially within the same crystalline phase.

2. Crystalline material of claim 1, which is a co-crystal containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and the coformer within the same crystalline phase in a molar ratio ranging from from 2:1 to 1:2, in particular from 1.5:1 to 1:1.5, and especially from 1.1:1 to 1:1.1.

3. Crystalline material according to claim 1 or 2 comprising a multicomponent molecular crystal (co-crystal) containing N-[2-[(4Z)-4-[[(4-fluorophenyl)methylamino]hydroxymethylidene]-1-methyl-5,6-dioxopyrimidin-2-yl]propan-2-yl]-5-methyl-1,3,4-oxadiazole-2-carboxamide potassium salt and cyclamic acid, exhibiting a X-ray powder diffraction pattern with the characteristic peaks, expressed in d-values (Å), 16.77 (vs), 13.89 (m), 6.95 (m), 4.21 (m); especially with the characteristic peaks, expressed in d-values (Å), 16.77 (vs), 13.89 (m), 10.68 (w), 8.07 (w), 7.48 (w), 6.95 (m), 6.43 (w), 5.69 (w), 5.34 (w), 4.21 (m).

4. Crystalline material according to claim 1 or 2 comprising a multicomponent molecu-lar crystal (co-crystal) containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and saccharin, exhibiting a X-ray powder diffraction pattern with the characteristic peaks, expressed in d-values (Å), 14.52 (vs), 13.93 (s), 10.70 (m), 6.96 (s); especially with the characteristic peaks, expressed in d-values (Å), 14.52 (vs), 13.93 (s), 10.70 (m), 8.10 (w), 7.52 (w), 6.96 (s), 6.42 (w), 4.47 (w), 4.40 (w), 4.10 (w).

5. Crystalline material according to claim 1 or 2 comprising a multicomponent molecular crystal (co-crystal) containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and stearic acid, exhibiting a X-ray powder diffraction pattern with the characteristic peaks, expressed in d-values (Å), 24.63 (s), 16.59 (vs), 15.39 (m), 9.99 (m); especially with the characteristic peaks, expressed in d-values (Å), 24.63 (s), 16.59 (vs), 15.39 (m), 12.48 (w), 9.99 (m), 8.37 (w), 7.16 (w), 5.39 (w), 5.20 (w), 4.54 (w).

6. Crystalline material according to claim 1 or 2 comprising a multicomponent molecular crystal (co-crystal) containing N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and succinic acid, exhibiting a X-ray powder diffraction pattern with the characteristic peaks, expressed in d-values (Å), 13.83 (vs), 10.64 (s), 9.16 (s), 6.94 (vs), 5.34 (m), 4.39 (m); especially with the characteristic peaks, expressed in d-values (Å), 13.83 (vs), 10.64 (s), 9.16 (s), 7.93 (w), 6.94 (vs), 6.40 (w), 5.69 (w), 5.34 (m), 4.46 (w), 4.39 (m).

7. Process for the preparation of a solid form according to any of claims 1 to 6, which process comprises the steps of
a) combining raltegravir potassium and the coformer in a suitable solvent,
b) agitating the mixture provided in step (a), and
c) separating the solid material and drying.

8. Pharmaceutical composition comprising the crystalline material or multicomponent molecular crystal according to any of claims 1 to 6 and a pharmaceutically acceptable carrier or diluent.

9. Pharmaceutical composition of claim 8 comprising N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt and the coformer within the same crystalline phase,
**characterized in that**
the coformer is cyclamic acid, and the composition exhibits at least one X-ray powder diffraction peak, expressed in d-values (Å), selected from the group consisting of 16.77, 13.89, 6.95, 4.21;
or the coformer is stearic acid, and the composition exhibits at least one X-ray powder diffraction peak, expressed in d-values (Å), selected from the group consisting of 24.63, 16.59, 15.39, 9.99;
or the coformer is saccharin, and the composition exhibits at least one X-ray powder diffraction peak, expressed in d-values (Å), selected from the group consisting of 14.52, 13.93, 10.70, 6.96;
or the coformer is succinic acid, and the composition exhibits at least one X-ray powder diffraction peak, expressed in d-values (Å), selected from the group consisting of 13.83, 10.64, 9.16, 6.94, 5.34, 4.39.

10. A therapeutic method for inhibiting HIV integrase, for the treatment or prophylaxis of HIV infection, or for the treatment, prophylaxis, or delay in the onset of AIDS, which method comprises administering to a subject in need of such therapy an effective amount of a crystalline material according to any of claims 1 to 6, or of a pharmaceutical composition according to any of claims 8 and 9.

11. A method of delivering a solid form of N-[2-[(4Z)-4-[[(4-fluorophenyl)methylamino]-hydroxymethylidene]-1-methyl-5,6-dioxopyrimidin-2-yl]propan-2-yl]-5-methyl-1,3,4-oxadiazole-2-carboxamide potassium salt, which method comprises administering to a host an effective amount of crystalline material according to any of claims 1 to 6, or of a pharmaceutical composition according to any of claims 8 and 9.

12. Use of a crystalline material according to any of claims 1 to 6, or of a pharmaceutical composition according to any of claims 8 and 9 for the manufacture of a medicament, especially a medicament useful for inhibiting HIV integrase, for the treatment or prophylaxis of HIV infection, or for the treatment, prophylaxis, or delay in the onset of AIDS.
